(19) Europäisches Patentamt
European Patent Office
Office européen des brevets

(11) Publication number: **0 046 653 B1**

# (12) EUROPEAN PATENT SPECIFICATION

(45) Date of publication of patent specification: **16.05.84**

(51) Int. Cl.$^3$: **C 07 C 17/18**

(21) Application number: **81303761.1**

(22) Date of filing: **18.08.81**

## (54) Method for converting carboxylic acid groups to trichloromethyl groups.

(30) Priority: **21.08.80 US 180064**

(43) Date of publication of application:
***03.03.82 Bulletin 82/09***

(45) Publication of the grant of the patent:
**16.05.84 Bulletin 84/20**

(84) Designated Contracting States:
**BE CH DE FR GB IT LI NL SE**

(56) References cited:
**Chemical Abstracts vol. 53, no. 9, 10 May 1959 Columbus, Ohio, USA, M.M. ROBISON "Chlorodehydroxylation of nitrogen heterocycles with phenylphosphonic dichloride", column 8155 a-e**

**Chemical Abstracts vol. 79, no. 5, 6 August 1973, Columbus, Ohio, USA, K. TAKAHASHI et al. "Conversion of carboxyl to a trichloromethyl group in reactions of imidazole- and pyridine-carboxylic acids with phosphorus pentachloride", page 451, column 2, abstract no. 31986m**

**The file contains technical information submitted after the application was filed and not included in this specification**

(73) Proprietor: **THE DOW CHEMICAL COMPANY
Dow Center 2030 Abbott Road Post Office Box 1967
Midland Michigan 48640 (US)**

(72) Inventor: **McKendry, Lennon Harold
5314 Campau Drive
Midland Michigan 48640 (US)**
Inventor: **Ricks, Michael John
3640 Serrano Avenue
Martinez Contra Costa California 94553 (US)**
Inventor: **Rogers, Richard Brewer
1837 Polk Street
Concord Contra Costa California 94520 (US)**

(74) Representative: **Allard, Susan Joyce et al
BOULT, WADE & TENNANT 27 Furnival Street
London EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 046 653 B1

**Description**

It is known to convert a ring attached carboxylic acid group to a trichloromethyl group when the acid group is on the ring of a nitrogen heteroaromatic compound and in a position adjacent to a ring nitrogen atom.

One reason for making such conversions is to allow for the preparation of compounds which cannot be prepared by the conventional practice of direct chlorination of methyl groups. For example, compounds containing alkoxy, alkyl or alkoxy or alkyl substituted aryl groups, in addition to the methyl group to be converted, cannot normally be prepared by direct chlorination without chlorination of the above groups.

Most known processes for the conversion of carboxylic acid groups to trichloromethyl groups involve treatment of the carboxylic acid compound with phosphorus pentachloride usually in the presence of excess thionyl chloride. Such processes are taught by Takahashi et al. "Kinetic study on the conversion of pyridine and Quinolinecarboxylic acids to the corresponding Trichloromethyl Compound" J. Het. Chem *15* 893 (1978); Chemical Abstracts *74*, 125566y; Chemical Abstracts *79* 31986m and Chemical Abstracts *81* 105395h. Related processes for other types of chlorinations are taught in Chemical Abstracts *63* 990ld and U.S. Patent 2,907,798.

Since the known procedures are only useful in the conversion of carboxylic acid groups to trichloromethyl groups when the acid group is adjacent to the nitrogen atom in a nitrogen heteroaromatic cyclic, it would be desirable to find less selective procedures and procedures which can be employed with other than nitrogen heteroaromatics.

This invention is directed to a method for the conversion of carboxylic acid groups to trichloromethyl groups which comprises contacting an aryl or N-heteroaryl compound containing a carboxylic acid group with a phenylphosphonic dichloride and phosphorus pentachloride.

Accordingly the present invention provides a method for converting a carboxylic acid group on the ring of an aryl or N-heteroaryl compound to a trichloromethyl group which comprises contacting an aryl or N-heteroaryl compound containing one or two carboxylic acid groups in non-sterically hindered ring positions with a phenylphosphonic dichloride and phosphorus pentachloride, provided that if an alkyl, alkylthio or alkoxy group is substituted on an aryl ring then a strong electron withdrawing group is also present on the ring.

This reaction can be characterized as follows:

$$R{-}(COOH)_n \;+\; \xrightarrow[\Delta]{(1)\ \phi{-}P(=O)Cl_2 \quad (2)\ PCl_5} \; R{-}(CCl_3)_n \;+\; \phi{-}P(=O)Cl_2 \;+\; POCl_3 \;+\; HCl$$

wherein R represents aryl or N-heteroaryl and n is 1 or 2. (No attempt has been made to present a balanced equation.)

The aryl or N-heteroaryl carboxylic acid compounds used in the present process are an aryl or N-heteroaryl compound containing one or two carboxylic acid groups in non-sterically hindered ring positions and are substantially chemically and physically stable under the acidic conditions of the reaction, with the exception of the conversion of the carboxylic acid groups to the corresponding trichloromethyl groups. When there are two acid groups present they should be in non-adjacent ring positions to avoid steric hindrance.

The compounds can in addition be ring substituted with additional sterically compatible groups which are non-reactive under the conditions of the reaction such as chloro, fluoro, bromo, nitro, cyano, alkyl alkoxy, alkylthio, hydroxyl, aryl, alkyl substituted aryl, alkoxy substituted aryl, aryloxy, trichloromethyl or trifluoromethyl. To further insure that problems of steric hindrance are avoided, it is important that no groups having an atom radius larger than the atom radius of a chlorine atom are adjacent to a carboxylic acid group. Further, if an alkyl, alkylthio or alkoxy group is substituted on an aryl ring, it is necessary that a strong electron withdrawing group such as nitro or cyano is also on the ring. This latter requirement is not necessary for N-heteroaromatic compounds.

In the present specification and claims, the term "sterically compatible" is employed to designate substituent groups which are not affected by steric hindrance as defined in "The Condensed Chemical Dictionary", 7th edition, Reinhold Publishing Co., New York, page 893 (1966) with definition is as follows:

> "steric hindrance. A characteristic of molecular structure in which the molecules have a spatial arrangement of their atoms such that a given reaction with another molecule is prevented or retarded in rate."

Steric hindrance may be further defined as compounds having substituents whose physical bulk does not require confinement within volumes insufficient for the exercise of their normal behaviour as discussed in "Organic Chemistry" D. J. Cram and G. Hammond, 2nd edition, McGraw-Hill Book Company, N.Y. page 215 (1964).

In carrying out the present process, the order of the addition of the reactants is not critical although in some situations, one mixing procedure may be more desirable than another. For example, if the reaction is to be run on a large scale, it is helpful to allow the carboxylic acid compound and the phenyl phosphonic dichloride compound to react for a period of time (until HCl evolution ceases) before adding the phosphorus pentachloride. This allows for more control of any initial foaming which might occur and more control of the exothermic nature of the reaction. In other situations, all the reactants can be mixed together.

After the reactants are mixed, the mixture is heated at reflux, 100°—250°C. The process is usually carried out at atmospheric pressure; however, the reaction goes well at pressures of from 0.1 to 10 atmospheres.

The reaction is usually completed in from 4 hours to 7 days, dependent upon the temperature, reactants and amounts of reactant. Upon the completion of the reaction, the reaction mixture is cooled and the phosphorus oxychloride byproduct is removed by distillation under reduced pressure. The desired product can be separated and recovered by conventional techniques such as by fractional distillation under reduced pressure or by solvent extraction after the reaction mixture was made basic with dilute base. The product can be purified, if desired, by conventional techniques such as by water washing, drying and recrystallization from a solvent such as benzene, hexane, methanol, chloroform, ether, cyclohexane or acetonitrile.

The phenylphosphonic dichlorides are usually present in an amount of from 0.1 to 20 molar equivalents of the phenylphosphonic dichloride per carboxylic acid group on the carboxylic acid compound. Preferably, from 1 to 10 molar equivalents of the phenylphosphonic dichloride per carboxylic acid group is employed.

Representative of those phenylphosphonic dichlorides which can be employed in the practice of the present invention include those corresponding to the formula

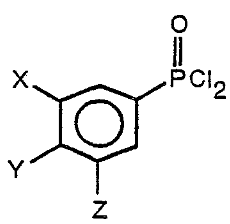

wherein X, Y and Z independently represents hydrogen, fluoro, chloro, cyano, trifluoromethyl, nitro and alkyl sulfonyl of 1 to 4 carbon atoms.

The phosphorus pentachloride is usually present in an amount of from 2 to 10 molar equivalents of the phosphorus pentachloride per carboxylic acid group on the carboxylic acid compound. Preferably, from 2 to 5 molar equivalents of the phosphorus pentachloride per carboxylic acid group are employed. If the carboxylic acid compound is ring substituted with a reactive group such a bromo, iodo or hydroxy group, an additional molar equivalent of the phosphorus pentachloride is necessary for each such group. These reactive groups are normally replaced with chlorine.

The following examples further illustrate the present invention.

Example 1

Preparation of 4-chloro-2,6-bis(trichloromethylpyridine).

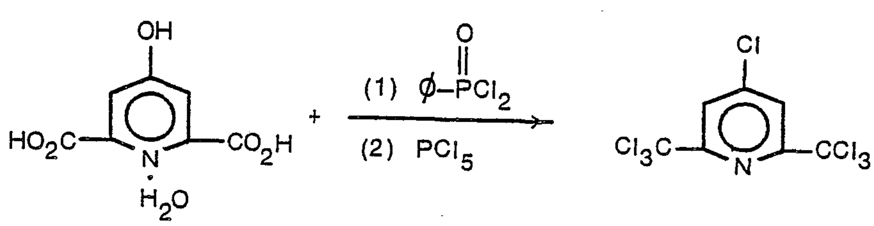


To a 5-litre flask equipped with a mechanical stirrer, thermometer and condenser was added 1950 g (10 moles) of phenylphosphonic dichloride. Thereafter 302 g (1.5 moles) of 2,6-dicarboxy-4-hydroxypyridine monohydrate (chelidamic acid monohydrate) was rapidly added. The mixture was slowly and carefully heated to 150°C over a two hour period. Thereafter, the mixture was cooled to 50°—60°C and 1029 g (5.25 moles) of phosphorus pentachloride was rapidly added. The mixture was

heated until the internal temperature was 75°—80°C and the heat source removed. The temperature exothermically rose to 125°C and after a short period, the temperature started to cool. When the reaction mixture reached a temperature of 90°C, a second portion of phosphorus pentachloride (1029 g (5.25 moles)) was added and the mixture heated at reflux (140°—145°C) for 12 hours. The condenser was replaced with a distilling head and the reaction mixture was distilled and the by-product phosphorus oxychloride was distilled off until a pot temperature of 215°C was reached. At this point, the distilling head was replaced with a short fractionating column and phenyl phosphonic dichloride was rapidly distilled off at 75°—85°C and 0.05 millimeters of mercury (mm Hg). The crude 4-chloro-2,6-bis(trichloromethyl)pyridine which remained solidified upon standing, and recrystallization of this material from methanol gave 314 g (60 percent of theoretical). The product melted at 101°—102°C and its structure was confirmed by its Nuclear Magnetic Resonance spectra (NMR)

In other runs, this compound was prepared in yields as high as 85 percent of theoretical.

Example 2

Preparation of 1-nitro-4-(trichloromethyl)benzene

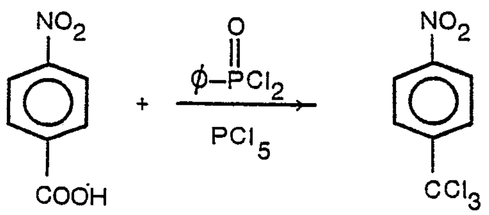


A mixture was prepared by adding with stirring and in the following order, 16.7 g (0.1 mole) of 4-nitrobenzoic acid, 15.7 g (0.11 mole) of phenylphosphonic dichloride and 52 g (0.25 mole) of phosphorus pentachloride. The mixture was heated and stirred and within 1 to 2 minutes, vigorous HCl evolution began and the mixture became a clear yellow liquid. The reaction mixture was heated at reflux for ~15 hours, cooled to 25°C and the phosphorus oxychloride byproduct was removed by evaporation under reduced pressure. The oil which remained as a residue was cautiously poured into a 10 percent solution of sodium carbonate in cold water and stirred vigorously until foaming ceased while maintaining the temperature below 30°C. The resultant solid crude 1-nitro-4-(trichloromethyl)benzene was recovered by filtration, washed with water, air dried and recrystallized from hexane to give 19.6 g (81 percent of theoretical) of product. The product melted at 44°—47°C and its structure was confirmed by NMR.

In other runs, this compound was prepared in yields as high as 94 percent of theoretical.

Example 3

Preparation of 2,3-dichloro-5-(trichloromethyl)pyridine

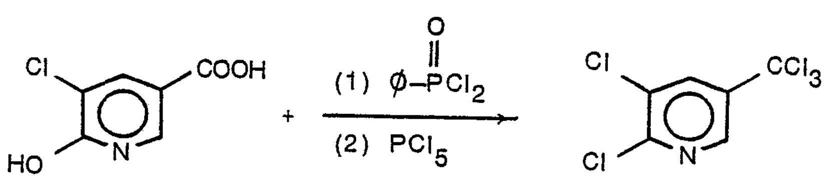


To a 5-liter flask equipped with an air stirrer, thermometer, and condenser was added 1000 g (5.13 moles) of phenylphosphonic dichloride. Thereafter, 383 g (2.707 moles) of 5-chloro-6-hydroxynicotinic acid (prepared by bubbling chlorine into a stirred aqueous suspension of 6-hydroxynicotinic acid) was added. The mixture was slowly heated and stirred over a 20 minute period, with the temperature rising to 73°C. The mixture was in the form of a thick paste.

To this mixture was slowly added 1755 g (8.4 moles) of phosphorus pentachloride over a 45 minute period. The hydrogen chloride by-product which formed was continuously removed and the heat was adjusted to maintain the temperature in the range of 83°—108°C. After the phosphorus pentachloride addition was complete, the mixture was heated to reflux and some of the phosphorus oxychloride by-product which formed was allowed to distill off. After a period of about 70 minutes, the temperature had exothermically risen to 169°C and the temperature was held in the range of 162°—180°C for 5¾ hours. During the above time additional phosphorus oxychloride was intermittently removed. The mixture was allowed to stand overnight and then poured over cracked ice, neutralized with 50 percent sodium hydroxide solution and the product extracted with hexane. The solvent was removed by evaporation under reduced pressure leaving 567 g of crude 2,3-dichloro-5-(trichloromethyl)pyridine.

The crude product was placed on a 15 tray vacuum jacketed Oldershaw distillation column and the light ends removed. The pot material was transferred to a Vigreux Claisen still and flash distilled to yield of 518 g (88.5 percent of theoretical) of a colorless oil which analyzed as 99 percent pure 2,3-dichloro-5-(trichloromethyl)pyridine. Upon analysis, the product was found to have carbon, hydrogen, chlorine and nitrogen contents of 26.99; 0.76; 66.49; and 5.23 percent, respectively, as compared with the theoretical contents of 27.15; 0.76; 66.81 and 5.28 percent, respectively, as calculated for the above named compound.

In Examples 1, 2 and 3, no attempt has been made to present balanced and complete reaction schemes.

By following the procedures as outlined in Examples 1, 2 and 3, the following compounds set forth below in Table 1 are prepared.

TABLE I

| Compound | Yield in Percent | Physical Property |
|---|---|---|
| 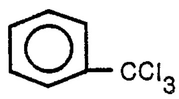 | 81 | $n_d^{20} = 1.5566$ |
| 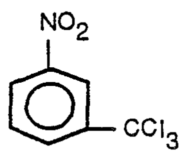 | 74 | $n_d^{25} = 1.5800$ |
| 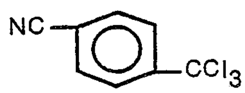 | 72 | $n_d^{25} = 1.5755$ |
| 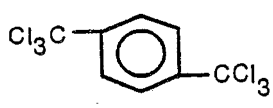 | 94 | M.P. = 104°–107°C |
| 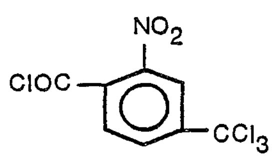 | 72 | M.P. = 209°–211°C as the anilide |
| 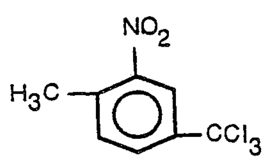 | 67 | $n_d^{25} = 1.5758$ |
| 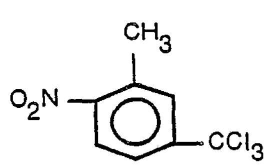 | 57 | $n_d^{25} = 1.5784$ |

TABLE I (Continued)

| Compound | Yield in Percent | Physical Property |
|---|---|---|
| 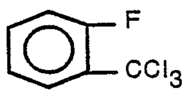 | 75 | $n_d^{25} = 1.5411$ |
| 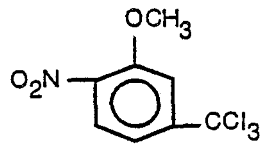 | 42 | $n_d^{25} = 1.5836$ |
| 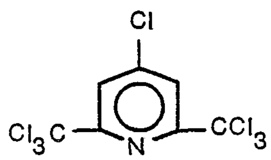 | 85 | M.P. = 101°–102°C |
| 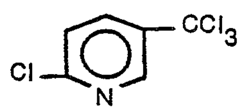 | 82 | M.P. = 52°–54°C |
| 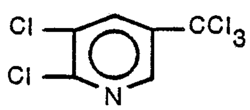 | 81 | $n_d^{25} = 1.5863$ |
| 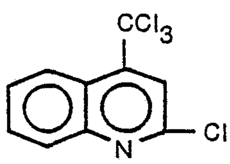 | 82 | M.P. = 67°–68°C |

The starting materials employed in the process of the present invention are known materials, the preparation of which is taught in the literature or they are articles of commerce.

**Claims**

1. A method for converting a carboxylic acid group on the ring of an aryl or N-heteroaryl compound to a trichloromethyl group which comprises contacting an aryl or N-heterary‌l compound containing one or two carboxylic acid groups in non-sterically hindered ring positions with a phenylphosphonic dichloride and phosphorus pentachloride, provided that if an alkyl, alkylthio or alkoxy group is substituted on an aryl ring then a strong electron withdrawing group is also present on the ring.

2. A method as claimed in claim 1 wherein the conversion is carried out at a temperature of from 100°C to 250°C.

3. A method as claimed in claim 1 or claim 2 wherein the phenylphosphonic dichloride is present in an amount of from 0.1 to 20 molar equivalents of the phenylphosphonic dichloride per carboxylic acid group to be converted.

4. A method as claimed in claim 3 wherein the amount is from 1 to 10 molar equivalents of the phenylphosphonic dichloride per carboxylic acid group.

5. A method as claimed in any one of the preceding claims wherein the phosphorus pentachloride is present in an amount of from 2 to 10 molar equivalents of the phosphorus pentachloride per carboxylic acid group to be converted.

6. A method as claimed in any one of the preceding claims wherein a carboxylic acid group on an aryl compound is converted.

7. A method as claimed in any one of claims 1 to 6 wherein a carboxylic acid group on a N-heteroaryl compound is converted.

8. A method as claimed in claim 6 wherein the aryl compound is ring substituted with at least one sterically compatible group from the group consisting of chloro, fluoro, bromo, nitro, cyano, alkyl, alkoxy, alkylthio, hydroxyl, aryl, alkyl substituted aryl, alkoxy substituted aryl, aryloxy or trifluoromethyl, with the proviso that when the substituent is alkyl, alkylthio or alkoxy, a strong electron withdrawing group is also present on the ring.

9. A method as claimed in claim 7 wherein the N-heteroaryl compound is ring substituted with at least one sterically compatible group from the group consisting of chloro, fluoro, bromo, nitro, cyano, alkyl, alkoxy, alkylthio, hydroxyl, aryl, alkyl substituted aryl, alkoxy substituted aryl, aryloxy or trifluoromethyl.

**Patentansprüche**

1. Verfahren zur Überführung einer an den Ring einer Aryl- oder N-Heteroaryl-Verbindung gebundenen Carboxyl-gruppe in eine Trichlormethylgruppe, dadurch gekenn-zeichnet, daß man eine Aryl- oder N-Heteroarylverbindung, die ein oder zwei Carboxylgruppen in nicht-sterisch gehinderten Ringpositionen enthält, mit einem Phenylphosphonsäuredichlorid und Phosphorpentachlorid in Kontakt bringt, mit der Maßgabe, daß bei Vorhandensein einer Alkyl-, Alkylthio- oder Alkoxygruppe als Substituent eines Arylringes ebenfalls eine starke Elektronen-anziehende Gruppe am Ring vorhanden ist.

2. Verfahren nach Anspruch 1, dadurch gekennzeichnet, daß die Überführung bei einer Temperatur von 100°C bis 250°C durchgeführt wird.

3. Verfahren nach Anspruch 1 oder 2, dadurch gekennzeichnet, daß das Phenylphosphonsäuredichlorid in einer Menge von 0,1 bis 20 Mol-Äquivalenten Phenylphosphonsäuredichlorid pro zu überführender Carboxylgruppe vorhanden ist.

4. Verfahren nach Anspruch 3, dadurch gekennzeichnet, daß die Menge 1 bis 10 Mol-Äquivalente Phenylphosphonsäuredichlorid pro Carboxylgrüppe beträgt.

5. Verfahren nach einem der vorhergehenden Ansprüche, dadurch gekennzeichnet, daß Phosphorpentachlorid in einer Menge von 2 bis 10 Mol-Äquivalenten Phosphorpentachlorid pro zu überführender Carboxylgruppe vorhanden ist.

6. Verfahren nach einem der vorhergehenden Ansprüche dadurch gekennzeichnet, daß eine Carboxylgruppe an einer Arylverbindung überführt wird.

7. Verfahren nach einem der Ansprüche 1 bis 6, dadurch gekennzeichnet, daß eine Carboxylgruppe an einer N-Heterarylverbindung überführt wird.

8. Verfahren nach Anspruch 6, dadurch gekennzeichnet, daß die Arylverbindung mit wenigstens einer sterisch kompatiblen Gruppe aus der Gruppe bestehend aus Chlor, Fluor, Brom, Nitro, Cyan, Alkyl, Alkoxy, Alkylthio, Hydroxyl, Aryl, alkylsubstituiertes Aryl, alkoxysubstituertes Aryl, Aryloxy oder Trifluormethyl am Ring substituiert ist, mit der Maßgabe, daß wenn dieser Substituent Alkyl, Alkylthio oder Alkoxy ist, ebenfalls eine stark Elektronen-anziehende Gruppe am Ring vorhanden ist.

9. Verfahren nach Anspruch 7, dadurch gekennzeichnet, daß die N-Heteroarylverbindung mit wenigstens einer sterisch kompatiblen Gruppe aus der Gruppe bestehend aus Chlor, Fluor, Brom, Nitro, Cyan, Alkyl, Alkoxy, Alkylthio, Hydroxyl, Aryl, alkylsubstituiertes Aryl, alkoxysubstituiertes Aryl, Arlyoxy oder Trifluormethyl am Ring substituiert ist.

**Revendications**

1. Procédé pour convertir un groupe acide carboxylique sur le noyau d'un composé arylique ou N-hétéro-arylique en un groupe trichlorométhylique, qui comporte la mise en contact d'un composé arylique ou N-hétéro-arylique contenant un ou deux groupes acid carboxylique sur des positions du noyau non encombrées stériquement, avec un dichlorure phénylphosphonique et du pentachlorure de phosphure, à condition que si un groupe alcoyle, alcoylthio ou alcoxy et substitué sur un noyau aryle, un groupe fort éliminant les électrons soit aussi présent sur le noyau.

2. Procédé selon la revendication 1, dans lequel on effectue la conversion à une température comprise entre 100°C et 250°C.

3. Procédé selon les revendications 1 ou 2, dans lequel le dichlorure phénylphosphonique est présent en une quantité comprise entre 0,1 et 20 équivalents molaires de dichlorure phénylphosphore par groupe acide carboxylique à convertir.

4. Procédé selon la revendication 3, dans lequel la quantité est comprise entre 1 et 10 équivalents molaires de dichlorure phénylphosphonique par groupe acide carboxylique.

5. Procédé selon l'une quelconque des revendications précédentes, dans lequel le pentachlorure de phosphore est présent en une quantité comprise entre 2 et 10 équivalents molaires de pentachlorure de phosphore par groupe acide carboxylique à convertir.

6. Procédé selon l'une quelconque des revendications précédentes, dans lequel un groupe acide carboxylique sur un composé arylique est converti.

7. Procédé selon l'une quelconque des revendications 1 à 6, dans lequel un groupe acide carboxylique sur un composé N-hétéro-arylique est converti.

8. Procédé selon la revendication 6, dans lequel le composé aryle est substitué sur le noyau avec au moins un groupe stériquement compatible choisi dans le groupe comprenant les groupes chloro, fluoro, bromo, nitro, cyano, alcoyle, alcoxy, alcoylthio, hydroxyle, aryle, aryle substitué par un reste alcoyle, aryle substitué par un reste alcoxy, aryloxy ou trifluorométhyle, à condition que, si le substituant est un groupe alcoyle, alcoylthio ou alcoxy, un groupe fort éliminant les électrons soit aussi présent sur le noyau.

9. Procédé selon la revendication 7, das lequel le composé N-hétéro-arylique est substitué sur le noyau avec au moins un groupe stériquement compatible choisi dans le groupe comprenant les groupes chloro, fluoro, bromo, nitro, cyano, alcoyle, alcoxy, alcoylthio, hydroxyle, aryle, aryle substitué par un reste alcoyle, aryle substitué par un reste alcoxy, aryloxy ou trifluorométhyle.